(19) **Europäisches Patentamt**

**European Patent Office**

**Office européen des brevets**

(11) **EP 1 145 707 A1**

(12) **EUROPEAN PATENT APPLICATION**
published in accordance with Art. 158(3) EPC

(43) Date of publication:
**17.10.2001 Bulletin 2001/42**

(21) Application number: **00901920.9**

(22) Date of filing: **27.01.2000**

(51) Int Cl.⁷: **A61K 7/42**, A61K 7/00

(86) International application number:
**PCT/JP00/00401**

(87) International publication number:
**WO 00/44341 (03.08.2000 Gazette 2000/31)**

(84) Designated Contracting States:
**AT BE CH CY DE DK ES FI FR GB GR IE IT LI LU MC NL PT SE**

(30) Priority: **28.01.1999 JP 2048999**
**25.03.1999 JP 8164399**
**19.01.2000 JP 2000010787**

(71) Applicant: **SHISEIDO COMPANY LIMITED**
**Tokyo 104-8010 (JP)**

(72) Inventors:
• **Iwai, Ichiro, Shiseido Res. Cen.(1)**
**Yokohama-shi, Kanagawa 223-8553 (JP)**

• **Hatao, Masato, Shiseido Res. Cen.(1)**
**Yokohama-shi, Kanagawa 223-8553 (JP)**
• **Yagi, Eiichiro, Shiseido Res. Cen.(1)**
**Yokohama-shi, Kanagawa 223-8553 (JP)**
• **Iwaki, Haruhi, Shiseido Res. Cen.(1)**
**Yokohama-shi, Kanagawa 223-8553 (JP)**
• **Matsuzaki, Fumiaki, Shiseido Res. Cen.(1)**
**Yokohama-shi, Kanagawa 223-8553 (JP)**

(74) Representative: **Henkel, Feiler, Hänzel**
**Möhlstrasse 37**
**81675 München (DE)**

(54) **COMPOSITIONS FOR EXTERNAL USE**

(57) Compositions for external use which contain a zinc compound, in particular, a composition for external use which contains a zinc compound and a thiol compound and a composition for external use which contains a higher fatty acid zinc salt as the zinc compounds and a polar oil derivative having an I.O.B. value of 0.1 or more. These compositions can promote the production of metallothionein in skin cells and thus prevent skin damage due to solar UV light, etc. without any problem of odor.

**EP 1 145 707 A1**

**Description**

Technical Field

The present invention relates to compositions for external use which can be used as, for example, external skin-treatment compositions.

Background Art

In modern life, the skin is exposed to various factors which can cause damage to skin cells. One typical example of such factors is solar UV rays.

Natural sunlight includes UV rays, visible rays, and IR rays. Of these rays, UV rays are known to induce damage to the skin. Sunburn is a type of acute inflammation caused by excessive exposure of the skin to UV rays. When the skin undergoes sunburn, epidermal cells are damaged, and sunburn cells; i.e., necrotized epidermal cells, are formed [Note: A sunburn cell is a cell with homogeneously eosinophilic cytoplasm and a vacuolated, dense nucleus which is clearly stained with hematoxylin, when subjected to hematoxylin-eosin (HE) staining (Hanada, *Nippi Kaishi* 106 (13), 1559-1561 (1996)). Sunburn cells represent one type among the most significant histological changes which occur on the epidermis upon irradiation with UV rays]. In many cases, sunburn results in pigmentation of the skin. As a result of the skin being chronically exposed to UV rays, aging of the skin is accelerated, and precancerous conditions of the skin may also result.

At present, in order to protect a living body from solar UV rays, compositions for external use (sunscreens) containing a UV absorbent or a UV-scattering agent and serving as shields against solar UV rays are widely used.

Because of ozone layer depletion and similar environmental phenomena, the necessity of protecting a living body from solar UV rays has been increasing.

As outlined above, there now exists a greater need to provide a far more effective means of protecting a living body from solar UV rays. Conventional sunscreens, when applied to the skin, have a function which enables them to block or absorb solar UV rays, to thereby prevent the UV rays from penetrating skin cells.

However, there has not yet been provided a composition for external use which enhances the protective action of a living body *per se* against solar UV rays and which has a function of satisfactorily preventing generation of the aforementioned sunburn cells.

One approach for protecting a living body from the solar UV rays which cause skin damage would be to find a substance having a function of eliminating the adverse effects caused, for example, by UV rays in skin cells, and to provide a composition for promoting production of the substance in the skin cells.

The body has mechanisms which protect it from harmful solar UV rays. For example, melanin contained in suntanned skin acts to scavenge free radicals, and thus is a useful UV-shielding substance. Urocanic acid contained in the skin acts to absorb solar UV rays, and is thought to protect the skin from solar UV rays through this absorption mechanism. The body also has enzymes which scavenge active oxygen, such as superoxide dismutase (SOD) and catalase; and antioxidants such as glutathione.

The present inventors have studied the aforementioned substances for protecting the skin from solar UV rays, and have come to draw attention to metallothionein, which is a metal-bound protein having a molecular weight of about 6,000 and is considered to be an intravital UV-shielding substance which acts antioxidatively against solar UV rays. Thus, the present inventors have considered that it may be possible to provide a novel composition for external use capable of preventing, by promoting the production of metallothionein in skin cells, skin damage caused by solar UV rays.

It is reported that metallothionein is derived from thionein, in a living body, by a specific heavy metal such as cadmium, zinc, copper, mercury, silver, or bismuth. Conventionally, in order to promote production of metallothionein in a living body, an agent for deriving metallothionein from thionein is administered orally, through intradermal injection, or intraperitoneally. A metallothionein derivative which can be used as a transdermal external composition has rarely been reported.

Disclosure of the Invention

The present inventors have focused on a variety of zinc compounds serving as substances for inducing production of metallothionein, and have studied means for utilizing such a zinc compound as an ingredient to be incorporated into compositions for external use.

In view of the foregoing, the present invention basically provides compositions for external use containing a zinc compound (hereinafter, the compositions may be referred to as "external use compositions of the present invention").

Best Mode for Carrying Out the Invention

No particular limitation is imposed on the zinc compound which may be incorporated into the external use compositions of the present invention, so long as the compound contains at least one zinc atom in the molecule, and does not raise any problem in terms of safety when incorporated into the external use compositions. Examples of the zinc compound which may be incorporated into the external use compositions include zinc gluconate, basic zinc carbonate, zinc chloride, higher fatty acid zinc salts (e.g., zinc laurate, zinc myristate, zinc palmitate, zinc stearate, zinc undecylenate, zinc caprate, zinc oleate, zinc rhodinate, zinc ricinoleate, and zinc neodecanoate), zinc para-phenolsulfonate, zinc pyrithion, zinc sulfate, zinc acetate, and zinc oxide.

Zinc compounds which are preferably selected and the amounts of the compounds incorporated into the external use compositions of the present invention vary with modes of the external use compositions.

The external use compositions of the present invention are roughly categorized into the following two modes:

I. composition containing a zinc compound and a thiol compound in combination (hereinafter the composition may be referred to as "external use composition A"); and

II. composition containing a higher fatty acid zinc salt serving as a zinc compound and polar oil derivatives having an I.O.B. value of 0.1 or more in combination (hereinafter the composition may be referred to as "external use composition B").

I. <u>External-use composition A</u>

As described above, external use composition A contains a zinc compound and a thiol compound.

The present inventors have considered that a thiol compound having an excellent antioxidative action and containing an SH group in the molecule can serve as an ingredient for preventing skin damage caused by solar UV rays.

However, when such a thiol compound is merely incorporated into an external use composition, the composition fails to satisfactorily exert the desired effect of preventing damage of skin cells.

In general, a thiol compound can be easily changed through, for example, oxidation by light, heat, or oxygen, and thus original functions of an external use composition containing a thiol compound (e.g., an antioxidative action attributed to the thiol compound) are easily lost. Therefore, an external use composition containing a thiol compound must be stored in a cool place, in an expensive light-shielding container, or in a low-oxygen-permeable hermetic container, or prepared immediately before use, in order to prevent decomposition of the thiol compound. These storage-related limitations stand as a great barrier against wide use of the external use composition containing the thiol compound.

As described above, stabilization of a thiol compound contained in an external use composition is usually difficult.

In addition, a thiol compound is generally accompanied by an offensive odor, and when a thiol compound is dissolved in an aqueous solution, a very bad odor derived from sulfur is generated. Therefore, the amount of a thiol compound which can be incorporated into an external use composition is limited.

The present inventors have extensively studied means for incorporating a thiol compound-which may be able to impart excellent skin-damage-resistance to skin cells-into an external use composition without raising the aforementioned problems.

As a result, the present inventors have found that, when a thiol compound and a zinc compound on which the inventors have focused are incorporated in combination into an external use composition, and the resultant composition is applied onto the skin, surprisingly, the resistance of skin cells to skin damage caused by solar UV rays can be synergistically enhanced.

Furthermore, the present inventors have found that, as described above, when a thiol compound and a zinc compound are incorporated in combination into an external use composition, quite surprisingly, the stability of the thiol compound in the external use composition is drastically enhanced, and the decomposition of the thiol compound by the action of light, heat, or oxygen and the generation of an offensive odor can be prevented.

Thus, the present inventors have attained the external use composition A.

It is thought that production of metallothionein, which is an SH-containing protein in a living body, is promoted by application of the external use composition A, and as a result, the resistance of the living body to oxidation stress can be enhanced, and skin damage caused by solar UV rays can be prevented.

Regardless of specific modes of the external use composition A, a thiol compound contained in the composition is stabilized.

Essential ingredients of the external use composition A

No particular limitation is imposed on the thiol compound which may be incorporated into the external use composition A, so long as the compound has an SH group in the molecule. Examples of the thiol compound which may be incorporated into the external use composition A include glutathione and derivatives thereof, such as glutathione, acetylated glutathione, glutathione hydrochloride, glutathione phosphate, and glutathione sulfate; cysteine and derivatives thereof, such as cysteine, N-acetylcysteine, cysteine hydrochloride, cysteine sulfate, and cysteine phosphate; mercaptoacetic acid; mercaptopropionic acid; ammonium thiolactate; and monoethanolamine thiolactate.

Such a thiol compound is appropriately selected and incorporated into the external use composition A in accordance with species of other ingredients to be incorporated. In general, from the viewpoints of ease of handling, safety, and effectiveness, glutathione, which is an amino-acid-type thiol compound, or a derivative thereof (hereinafter may collectively be referred to as "glutathiones"), or cysteine, which is also an amino-acid-type thiol compound, or a derivative thereof (hereinafter may be collectively referred to as "cysteines") is preferably incorporated into the external use composition A. More preferably, glutathione or N-acetylcysteine is incorporated into the composition A.

The amount of a thiol compound incorporated into the external use composition A is not particularly limited, and the amount may be appropriately varied in accordance with specific use of the composition, the product form of the composition, or species of other ingredients to be incorporated into the composition. The amount of a thiol compound incorporated into the external use composition A is preferably 0.001-20.0 wt.%, more preferably 0.1-20.0 wt.%, much more preferably 1.0-20.0 wt.%, based upon the total of the composition. Specific one thiol compound, or if necessary, a combination of two or more thiol compounds may be incorporated into the external use composition A.

No particular limitation is imposed on the zinc compound which may be incorporated into the external use composition A, so long as the compound contains at least one zinc atom in the molecule, and does not raise any problem in terms of safety when incorporated into the composition. Examples of the zinc compound which may be incorporated into the external use composition A include zinc gluconate, basic zinc carbonate, zinc chloride, zinc laurate, zinc myristate, zinc palmitate, zinc para-phenolsulfonate, zinc pyrithion, zinc stearate, zinc sulfate, zinc undecylenate, zinc acetate, zinc rhodinate, zinc ricinoleate, zinc neodecanoate, and zinc oxide.

The amount of a zinc compound incorporated into the external use composition A is not particularly limited, and the amount may be appropriately varied in accordance with specific use of the composition, the product form of the composition, or species of other ingredients to be incorporated into the composition. The amount of a zinc compound incorporated into the external use composition A is preferably 0.01-20.0 wt.%, more preferably 0.05-10.0 wt.%, based upon the total of the composition. When the amount of a zinc compound is less than 0.01 wt.% based upon the total of the external use composition, intended stabilization of a thiol compound contained in the external use composition A is not satisfactorily enhanced. Therefore, particularly when the external use composition A is used for shielding solar UV rays, the composition may fail to exert the intended UV ray-shielding effect satisfactorily. In contrast, when the amount of a zinc compound incorporated into the external use composition A is in excess of 20.0 wt.% based upon the total of the composition, the zinc compound may adversely affect the stability of the base.

Specific one zinc compound, or if necessary, a combination of two or more zinc compounds may be incorporated into the external use composition A.

As described above, when a thiol compound and a zinc compound are incorporated in combination into the external use composition A, the composition can be used as an "external use composition for preventing skin damage," which protects a living body from solar UV rays which cause skin damage, in skin cells *per se.*

When the external use composition A is applied onto the skin, production of metallothionein in skin cells, which is a protective mechanism inherent to a living body, is enhanced, and skin damage caused by exposure of the skin to UV rays can be prevented. In addition, production of sunburn cells in the epidermis can be prevented, to thereby prevent sunburn.

Furthermore, by the action of a zinc compound, the stability of an originally unstable thiol compound in an external use composition can be drastically enhanced. Therefore, strict control-as is conventionally require-is no longer required for the external use composition A; i.e., the composition is not necessarily stored in a light-shielding container, in a low-oxygen-permeable container, or in a hermetic container. In addition, the amount of a thiol compound incorporated into the composition can be varied in accordance with the intended use of the composition. Even when storage of the external use composition A is not strictly managed, significant loss of the excellent antioxidative action of a thiol compound does not occur over time. Moreover, even when a large amount of a thiol compound is incorporated into the composition, the composition is not accompanied by an offensive odor.

Irrespective of the reason for incorporating a thiol compound into the external use composition A [for example, the thiol compound is considered to exert (1) the effect of preventing aging of the skin with aging or (2) the effect of preventing skin stress due to particles in air (e.g., cigarette smoke or exhaust gas), in addition to the effect of protecting the body from solar UV rays; e.g., preventing oxidation damage in the skin], the stability of the thiol compound in the external use composition is drastically enhanced, and range of use of the composition is remarkably broadened.

The external use composition A may contain an antioxidant in addition to the aforementioned essential ingredients, to thereby further enhance the stability of a thiol compound. Examples of such an antioxidant include BHT (dibutylhydroxytoluene), BHA (butylhydroxyanisol), gallates, and NDGA (nordihydroguaiaretic acid). Of these, BHT or BHA is preferable.

The amount of an antioxidant incorporated into the external use composition A is not particularly limited, since the amount may be varied in accordance with the amount of each of the aforementioned essential ingredients; i.e., a thiol compound and zinc oxide, and the actual mode of incorporation. The amount of an antioxidant incorporated into the external use composition is preferably 0.001-10.0 wt.%, more preferably 0.01-2.0 wt.%, based upon the total of the composition.

II. External-use composition B

As described above, the external use composition B contains a higher fatty acid zinc salt and a polar oil derivative having an I.O.B. value of 0.1 or more.

Although the effect obtained by inducing metallothionein has been mentioned, a metallothionein-inducing agent which is actually usable as a transdermal external agent has rarely been reported, as described above, for the reasons described below.

Cadmium chloride or zinc chloride, which has been conventionally known as a metallothionein-inducing agent, is usually water-soluble, and thus the transdermal penetrability of such an agent is very low. Zinc acetate, which is known only as an transdermal metallothionein-inducing agent (A. J. MORGAN, G. LEWIS, W. E. VAN DEN HOVEN, AND P. J. AKKERBOOM, British Journal of Dermatology (1993) 129, 563-570), encounters difficulty in being used as a transdermal metallothionein-inducing agent, since it generates an offensive odor.

The present inventors have focused on a higher fatty acid zinc salt, which may be able to induce production of metallothionein in a living body when administered transdermally, and does not raise any problem in terms of odor. However, since the solubility of a higher fatty acid zinc salt is very low with respect to a usual water-soluble or oil-soluble solvent, the higher fatty acid zinc salt encounters difficulty in being absorbed transdermally.

In order to solve such problems, the present inventors have found that, when a higher fatty acid zinc salt is mixed with polar oil derivatives having an I.O.B. value of 0.1 or more, the salt is dissolved in the derivative, and consequently, the higher fatty acid zinc salt can be absorbed transdermally, and the level of metallothionein produced in skin cells is remarkably enhanced. The present inventors have also found that, when a higher fatty acid zinc salt is mixed with polar oil derivatives having an I.O.B. value of 0.1 or more and the resultant mixture is administered transdermally, production of sunburn cells in the epidermis through irradiation of solar UV rays is considerably suppressed, as compared with the case in which only a methoxycinnamic acid derivative having a UV-absorbing action is administered transdermally.

As used herein, I.O.B. (abbreviation of Inorganic Organic Balance) value signifies an index representing the polarity of an oily ingredient. Briefly, I.O.B. value represents the ratio of an inorganic value of an oily ingredient to an organic value of the oily ingredient [note: the ratio is calculated as follows: an organic value of 20 is assigned for each carbon atom in the molecule of the oily component, and an inorganic value of 100 is assigned per hydroxy group in the molecule of the oily component, and these values are used as yardsticks for calculation of an inorganic value of another substituent (inorganic group); see (1) "Organic Analysis" authored by Fujita (1930), published by Kania Shoten, (2) "Prediction of Organic Compounds and Organic Conceptual Diagram ("Kagaku-no-Ryoiki 11-10" (1957), authored by Fujita, (3) "Systematic Organic Qualitative Analysis (Book of Purified Substances)" (1970), p 487, authored by Fujita and Akatsuka, published by Kazama Shoten, (4) "Organic Conceptual Diagram, Its Fundamentals and Applications" (1984), p 227, authored by Koda, published by Sankyo Shuppan, (5) "Design of Emulsion Formulations by use of Organic Conceptual Diagram" (1985), p 98, authored by Yaguchi, published by Nippon Emulsion K.K., and (6) R. H. Ewell, J. M. Harrison, and L. Berg: Ind. Eng. Chem. 36, 871 (1944)], and is specifically expressed by:

$$\text{I.O.B.} = <\text{inorganic value of the oily ingredient}> \div <\text{organic value of the oily ingredient}>.$$

The greater the I.O.B. value, the more prominent the inorganic properties of the oil ingredient, indicating higher polarity.

Essential ingredients of the external use composition B

The external use composition B contains polar oil derivatives having an I.O.B. value of 0.1 or more (hereinafter simply referred to as "polar oil derivative," unless otherwise specified).

When the I.O.B. value of polar oil derivatives is 0.1 or more, the derivatives can easily dissolve a higher fatty acid zinc salt serving as the other essential ingredient of the external use composition B, and thus the transdermal penetrability of the zinc salt can be enhanced. In contrast, when the I.O.B. value of a polar oil derivative is less than 0.1, polarity of the derivative is lowered, and the derivative may fail to dissolve a higher fatty acid zinc salt satisfactorily.

No particular limitation is imposed on the polar oil derivative which may be incorporated into the external use composition B, so long as the derivative satisfies conditions in terms of I.O.B. value (i.e., 0.1 or more), and is oil derivatives which are used in usual external use compositions (e.g., cosmetic compositions), for example, an oil ester derivative, an oil carboxyl derivative, or an oil carboxylic acid salt.

Preferred examples of the polar oil derivatives include para-methoxycinnamic acid derivatives such as ethyl para-methoxycinnamate, isopropyl para-methoxycinnamate, 2-ethoxyethyl para-methoxycinnamate, 2-ethylhexyl para-methoxycinnamate (also called octyl para-methoxycinnamate), sodium para-methoxycinnamate, potassium para-methoxycinnamate, and glyceryl octanoate di-para-methoxycinnamate. Such a para-methoxycinnamic acid derivative exerts a UV-shielding effect, and effectively suppresses generation of sunburn cells. Examples of the polar oil derivative which may be incorporated into the external use composition B also include di-2-ethylhexyl succinate, pentaerythritol tetra-2-ethylhexanoate, glyceryl tri-2-ethylhexanoate, cetyl octanoate, isopropyl myristate, isopropyl palmitate, butyl stearate, hexyl laurate, myristyl myristate, decyl oleate, hexyldecyl dimethyloctanoate, cetyl lactate, myristyl lactate, isocetyl stearate, cholesteryl 12-hydroxystearate, ethylene glycol di-2-ethylhexanoate, neopentyl glycol dicaprate, diisostearyl malate, glyceryl di-2-heptylundecanoate, trimethylolpropane tri-2-ethylhexanoate, trimethylolpropane triisostearate, cetyl 2-ethylhexanoate, 2-ethylhexyl palmitate, glyceryl trimyristate, glyceryl tri-2-heptylundecanoate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, di-2-hexyldecyl adipate, diisopropyl sebacate, ethyl acetate, butyl acetate, amyl acetate, and triethyl citrate.

Such polar oil derivatives are appropriately selected and incorporated into the external use composition B in accordance with specific use of the composition or species of other ingredients to be incorporated into the composition. From the viewpoints of ease of handling, safety, and effectiveness, 2-ethylhexyl para-methoxycinnamate, which is one of the aforementioned para-methoxycinnamic acid derivatives, is preferably incorporated into the external use composition B.

The amount of polar oil derivatives incorporated into the external use composition B is not particularly limited, and the amount may be appropriately varied in accordance with specific use of the composition, the product form of the composition, or species of other ingredients to be incorporated into the composition. The amount of the polar oil derivatives incorporated into the external use composition B is preferably 0.01-30.0 wt.%, more preferably 0.1-30.0 wt.%, much more preferably 1.0-30.0 wt.%, based upon the total of the composition.

Specific one polar oil derivative, or if necessary, a combination of two or more polar oil derivatives may be incorporated into the external use composition B.

A higher fatty acid zinc salt must be incorporated into the external use composition B together with a polar oil derivative.

No particular limitation is imposed on the higher fatty acid zinc salt which may be incorporated into the external use composition B, so long as the zinc salt does not raise any problem in terms of safety when incorporated into the composition. Specifically, the higher fatty acid zinc salt is a zinc salt of a fatty acid having 6 or more carbon atoms. The number of the carbon atoms is preferably 8-20. When the number of the carbon atoms is less than 8 or in excess of 20, the solubility of the higher fatty acid zinc salt with respect to the polar oil derivative tends to lower.

Preferred examples of the higher fatty acid zinc salt which may be incorporated into the external use composition B include zinc laurate, zinc myristate, zinc palmitate, zinc stearate, zinc undecylenate, zinc caprate, zinc oleate, zinc rhodinate, zinc ricinoleate, and zinc neodecanoate.

The amount of a higher fatty acid zinc salt incorporated into the external use composition B is not particularly limited, and the amount may be appropriately varied in accordance with specific use of the composition, the product form of the composition, or species of other ingredients to be incorporated into the composition. The amount of a higher fatty acid zinc salt incorporated into the external use composition B is preferably 0.01-20.0 wt.%, more preferably 0.05-10.0 wt.%, based upon the total of the composition.

When the amount of a higher fatty acid zinc salt is less than 0.01 wt.% based upon the total of the external use composition B, the composition may fail to exert the intended effect of preventing skin damage satisfactorily. In contrast, when the amount of a higher fatty acid zinc salt is in excess of 20.0 wt.% based upon the total of the composition, the zinc salt may adversely affect the stability of the base.

Specific higher fatty acid zinc salts may be incorporated into the external use composition B singly, or if necessary, in combination of two or more species.

As described above, when polar oil derivatives and a higher fatty acid zinc salt are incorporated in combination into the external use composition B, the originally insoluble higher fatty acid zinc salt is dissolved in the polar oil derivative, and the transdermal penetrability of the zinc salt can be enhanced. Therefore, the external use composition B can be used as an "external use composition for preventing skin damage," which protects a living body from solar UV rays which cause skin damage, in skin cells *per se.*

When the external use composition B is applied onto the skin, production of metallothionein in skin cells, which is a protective mechanism inherent to a living body, is enhanced, and skin damage caused by exposure of the skin to solar UV rays can be prevented. In addition, production of sunburn cells in the epidermis can be prevented, to thereby prevent sunburn. Particularly when a para-methoxycinnamic acid derivative is chosen as polar oil derivatives, skin damage caused by solar UV rays can be synergistically prevented, since the derivative can also shield the skin from the solar UV rays.

The external use composition B is suitable for transdermal administration, since the composition does not raise any problem in terms of odor.

III. Specific modes of the external use compositions of the present invention

In addition to the aforementioned essential ingredients, the external use compositions (both cases of composition A and composition B) of the present invention may contain-in amounts that do not impede the effects of the present invention-ingredients that exhibit pharmaceutical effects, such as a humectant, a whitening agent, an antiinflammatory agent, an activator, a blood flow stimulant, an antiseborrheic agent, a plant extract, a variety of vitamins, and a UV-shielding agent, and other ingredients which can be generally incorporated into external use compositions.

Particularly, when the external use compositions are intended to be used exclusively for providing protection against solar UV rays, incorporation of UV-shielding agents provides remarkable improvement in protective effect against solar UV rays. (Note that, in connection with the external use compositions B of the present invention, a UV absorbent having an I.O.B. value of 0.1 or more (such as p-methoxycinnamic acid derivatives) to be incorporated therein as an essential ingredient is excluded from the category of general ingredients that form the external use compositions B.)

Examples of UV absorbents include p-aminobenzoic-acid-based UV absorbents such as p-aminobenzoic acid, monoglyceryl p-aminobenzoate, ethyl p-N,N-dipropoxy-aminobenzoate, ethyl p-N,N-diethoxy-aminobenzoate, ethyl p-N,N-dimethyl-aminobenzoate, and butyl p-N,N-dimethyl-aminobenzoate; anthranilic-acid-based UV absorbents such as homomenthyl N-acetylanthranilate; salicylic-acid-based UV absorbents such as amyl salicylate, menthyl salicylate, homomenthyl salicylate, octyl salicylate, phenyl salicylate, benzyl salicylate, p-isopropanolphenyl salicylate;

cinnamic-acid-based UV absorbents such as octyl cinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl $\alpha$-cyano-$\beta$-phenylcinnamate, 2-ethylhexyl $\alpha$-cyano-$\beta$-phenylcinnamate, glyceryl octaoate di-p-methoxycinnamate, and 3-methyl-4-[methylbis(trimethylsiloxy)silyl]butyl 3,4,5-trimethoxycinnamate;

benzophenone-based UV absorbents, such as 2,4-dihydroxybenzophenone, 2,2'-dihydroxy-4-methoxybenzophenone, 2,2'-dihydroxy-4,4'-dimethoxybenzophenone, 2,2'4,4'-tetrahydroxybenzophenone, 2-hydroxy-4-methoxybenzophenone, 2-hydroxy-4-methoxy-4'-methylbenzophenone, 2-hydroxy-4-methoxy-4'-methylbenzophenone, a 2-hydroxy-4-methoxybenzophenone-5-sulfonic acid salt, 4-phenylbenzophenone, 2-ethylhexyl-4'-phenylbenzophenone-2-carboxylate, 2-hydroxy-4-n-octyloxybenzophenone, and 4-hydroxy-3-carboxybenzophenone; and other UV absorbents such as 3-(4'-methylbenzylidene)-d,1-camphor, 3-benzylidene-d,1-camphor, urocanic acid, ethyl urocanate, 2-phenyl-5-methylbenzoxazole, 2-(2'-hydroxy-5-methylphenyl)benzotriazole, 2-(2'-hydroxy-5'-t-octylphenyl)benzotriazole, 2-(2'-hydroxy-5'-methylphenyl)benzotriazole, dibenzalazine, dianisoylmethane, 4-t-butyl-4'-methoxydibenzoylmethane, and 5-(3,3-dimethyl-2-norbornylidene)-3-pentan-2-one.

Examples of UV-blockers include generally employed powder ingredients exhibiting excellent UV-blocking properties, such as titanium dioxide.

In the present invention, the term "external use composition" refers to any of cosmetic compositions, drug compositions, quasi drug compositions, and similar compositions, which are applied to outer skin. The external use composition may take a wide variety of forms, including an aqueous solution, a solubilized system, an emulsion, a powder, an oil, a gel, an ointment, an aerosol, a water-oil two-layered system, and a water-oil-powder three-layered system. For example, with respect to skincare cosmetic compositions, a variety of products such as face washes, lotions, milky lotions, creams, gels, essences (beauty lotions), packs and masks, etc. may be mentioned, and they may be available in any of the aforementioned forms. Also, makeup cosmetic compositions may be available in product forms including foundations. Moreover, in the case of drug or quasi-drug compositions, they may take ointment form or other wide variety of product forms. Thus, no limitation is imposed on the form and product type of the external use compositions of the present invention.

The external use compositions of the present invention can be prepared through a conventional method by

EP 1 145 707 A1

adding, in accordance with the form and product type of interest, the aforementioned essential ingredients and optional pharmaceutically active ingredients to a known base ingredient which is generally employed in the manufacture of external use compositions, in such amount that do not impede the intended effects of the present invention.

The following ingredients may be incorporated as needed:

liquid oils and fats such as avocado oil, camellia oil, evening primrose oil, turtle oil, macadamia nut oil, corn oil, mink oil, olive oil, rape seed oil, egg yolk oil, sesame oil, persic oil, wheat germ oil, sasanqua oil, castor oil, linseed oil, safflower oil, cotton seed oil, perilla oil, soybean oil, peanut oil, tea seed oil, *kaya* oil, rice bran oil, Chinese tung oil, Japanese tung oil, jojoba oil, germ oil, triglycerin, glyceryl trioctanoate, and glyceryl triisopalmitate;

solid oils and fats such as cacao butter, coconut oil, horse fat, hydrogenated coconut oil, palm oil, beef tallow, mutton tallow, hydrogenated beef tallow, palm kernel oil, lard, cow bone fat, japan wax kernel oil, hydrogenated oil, beef foot oil, japan wax, and hydrogenated castor oil;

waxes such as beeswax, candelilla wax, cotton wax, carnauba wax, bayberry wax, insect wax, spermaceti, montan wax, rice bran wax, lanolin, kapok wax, lanolin acetate, liquid lanolin, sugar cane wax, a lanolin fatty acid isopropyl ester, hexyl laurate, hydrogenated lanolin, jojoba wax, hard lanolin, shellac wax, POE lanolin alcohol ether, POE lanolin alcohol acetate, POE cholestanol ether, polyethylene glycol lanolin fatty acid, and POE hydrogenated lanolin alcohol ether; hydrocarbon oils such as liquid paraffin, ozokerite, squalene, pristane, paraffin, ceresine, squalane, vaseline, and microcrystalline wax;

higher fatty acids such as lauric acid, myristic acid, palmitic acid, stearic acid, behenic acid, oleic acid, 12-hydroxystearic acid, undecylenic acid, tall oil acid, isostearic acid, linoleic acid, linolenic acid, eicosapentaenoic acid (EPA), and docosahexaenoic acid (DHA);

higer alcohols including linear chain alcohols such as lauryl alcohol, cetyl alcohol, stearyl alcohol, behenyl alcohol, myristyl alcohol, oleyl alcohol, and cetostearyl alcohol; and branched chain alcohols such as monostearyl glyceryl ether (batyl alcohol), 2-decyltetradecanol, lanolin alcohol, cholesterol, phytosterol, hexyldodecanol, isostearyl alcohol, and octyldodecanol;

synthetic ester oils having an I.O.B. value of less than 0.1 or a non-determined I.O.B. value (such a synthetic ester oil may be incorporated, as a general ingredient, into the external use composition A or B), such as octyldodecyl myristate, lanolin acetate, isocetyl isostearate, dipentaerythrite fatty acid ester, n-alkylene glycol monoisostearate, methyl castor oil fatty acid ester, oleyl oleate, 2-heptylundecyl palmitate, and 2-hexyldecyl palmitate;

Synthetic ester oils having an I.O.B. value of 0.1 or more (such a synthetic ester oil may be incorporated, as a general ingredient, into the external use composition A), such as di-2-ethylhexyl succinate, pentaerythrite tetra-2-ethylhexanoate, glyceryl tri-2-ethylhexanoate, cetyl octanoate, isopropyl myristate, isopropyl palmitate, butyl stearate, hexyl laurate, myristyl myristate, decyl oleate, hexyldecyl dimethyloctanoate, cetyl lactate, myristyl lactate, isocetyl stearate, cholesteryl 12-hydroxystearate, ethylene glycol di-2-ethylhexanoate, neopentyl glycol dicaprate, diisostearyl malate, glyceryl di-2-heptylundecanoate, trimethylolpropane tri-2-ethylhexanoate, trimethylolpropane triisostearate, cetyl 2-ethylhexanoate, 2-ethylhexyl palmitate, gryceryl trimyristate, gryceryl tri-2-heptylundecanoate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, di-2-hexyldecyl adipate, diisopropyl sebacate, ethyl acetate, butyl acetate, amyl acetate, and triethyl citrate;

silicones including chain polysiloxanes such as dimethylpolysiloxane, methylphenylpolysiloxane, and methyl hydrogen polysiloxane; cyclic polysiloxanes such as decamethylpolysiloxane, dodecamethylpolysiloxane, and tetramethyl tetrahydrogen polysiloxane; silicone resins having a three-dimensional network structure; and silicone rubbers;

anionic surfactants including fatty acid soaps such as soap bar, sodium laurate, and sodium palmitate; higher-alkyl sulfuric acid ester salts such as sodium lauryl sulfate and potassium lauryl sulfate; POE alkyl ether sulfuric acid ester salts such as triethanolamine POE laurylether sulfate and sodium POE laurylether sulfate; N-acylsarcosine salts such as sodium N-lauroyl sarcosinate; higher fatty acid amido sulfonic acid salts such as sodium N-myristoyl-N-methyl taurate, sodium N-cocoyl-N-methyl taurate, and sodium N-lauryl-N-methyl taurate; POE alkyl ether phosphoric acid salts such as sodium POE oleylether phosphate and sodium POE stearylether phosphate; sulfosuccinic acid salts such as sodium di(2-ethylhexyl) sulfosuccinate, disodium mono(lauroylethanolamide polyoxyethylene) sulfosuccinate, and sodium lauryl polypropylene glycol sulfosuccinate; alkylbenzenesulfonates such as sodium linear dodecylbenzenesulfonate, triethanolamine linear dodecylbenzenesulfonate, and linear dodecylbenzenesulfonic acid; N-acylglutamates such as sodium N-lauroylglutamate, disodium N-stearoylglutamate, and sodium N-myristoyl-L-glutamate; higher fatty acid ester sulfonic acid ester salts such as sodium hydrogenated glyceryl cocoate sulfate; sulfonated oils such as Turkey red oil; POE alkyl ether carboxylates; POE alkylallyl ether carboxylates; α-olefin sulfonates; higher fatty acid ester sulfonic acid salts; secondary alcohol sulfuric acid ester salts;

higher fatty acid alkylolamide sulfuric acid ester salts; sodium lauroyl monoethanolamide succinate; ditriethanolamine N-palmitoylaspartate; and sodium caseinate;

cationic surfactants including alkyltrimethylammonium salts such as stearyltrimethylammonium chloride and lauryltrimethylammonium chloride; dialkyldimethylammonium salts such as distearyldimethylammonium chloride; alkylpyridinium salts such as poly(N,N'-dimethyl-3,5-methylenepiperidinium) chloride and cetylpyridinium chloride; alkyl quaternary ammonium salts; alkyldimethylbenzylammonium salts; alkylisoquinolinium salts; dialkylmorpholinium salts; POE alkylamines; alkylamine salts; polyamine fatty acid derivatives; amyl alcohol fatty acid derivatives; benzalkonium chloride; and benzethonium chloride;

amphoteric surfactants including imidazoline amphoteric surfactants such as sodium 2-undecyl-N,N,N-(hydroxyethylcarboxymethyl)-2-imidazoline and 2-cocoyl-2-imidazoliniumhydroxide-1-carboxyethyloxy disodium salt; and betaine amphoteric surfactants such as 2-heptadecyl-N-carboxymethyl-N-hydroxyethylimidazolinium betaine, lauryldimethylamino acetic acid betaine, alkylbetaine, amidobetaine, and sulfobetaine; and olephilic nonionic surfactants including sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglyceryl sorbitan penta-2-ethylhexanoate, and diglyceryl sorbitan tetra-2-ethylhexanoate; glycerin fatty acid esters such as cotton seed oil glyceride, glyceryl monoerucate, glyceryl sesquioleate, glyceryl monostearate, glyceryl monopyroglutamate monooleate, and glyceryl stearate malate; propylene glycol fatty acid esters such as propylene glycol monostearate; hydrogenated castor oil derivatives; glycerin alkylethers; and polyoxyethylene · methylpolysiloxane copolymers;

hydrophilic nonionic surfactants including POE sorbitan fatty acid esters such as POE sorbitan monooleate, POE sorbitan monostearate, and POE sorbitan tetraoleate; POE sorbitol fatty acid esters such as POE sorbitol monolaurate, POE sorbitol monooleate, POE sorbitol pentaoleate, and POE sorbitol monostearate; POE glycerin fatty acid esters such as POE glycerin monostearate, POE glycerin monoisostearate, and POE glycerin triisostearate; POE fatty acid esters such as POE monooleate, POE distearate, and POE dioleate; POE alkyl ethers such as POE lauryl ether, POE oleyl ether, POE stearyl ether, POE behenyl ether, POE 2-octyldodecyl ether, and POE cholesteryl ether; POE alkylphenyl ethers such as POE octylphenyl ether, POE nonylphenyl ether, and POE dinonylphenyl ether; Pluronic® surfactants such as Pluronic® ; POE·POP alkyl ethers such as POE·POP cetyl ether, POE·POP 2-decyltetradecyl ether, POE·POP monobutyl ether, POE·POP hydrogenated lanolin, and POE·POP glycerin ether; tetra POE·tetra POP ethylenediamine condensation products such as Tetronic® ; POE castor oil derivatives and POE hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamate monoisostearate diester, and POE hydrogenated castor oil maleate; POE beeswax or POE lanolin derivatives such as beeswax derivatives of POE sorbitol; alkanolamides such as coconut fatty acid diethanolamide, lauric acid monoethanolamide, and fatty acid isopropanolamide; POE propylene glycol fatty acid esters; POE alkylamines; POE fatty acid amides; sucrose fatty acid esters; POE nonylphenyl formaldehyde condensation products; alkylethoxydimethylamine oxides; and trioleyl phosphate.

preservatives such as methyl paraben, ethyl paraben, and butyl paraben;

sequestering agents such as sodium edetate and EDTA;

natural water-soluble polymers including plant polymers such as gum arabi, tragacanth gum, galactan, guar gum, carob gum, gum karaya, carageenan, pectin, agar, quinceseed (quince), algae colloid (brown algae extract), starch (rice, corn, potato, wheat), and glycyrrhizinic acid; microbial polymers such as xanthane gum, dextran, succinoglucan, and pullulan; animal polymers such as collagen, casein, albumin, and gelatin;

semi-synthetic water-soluble polymers including starch polymers such as carboxylmethyl starch and methylhydroxypropyl starch; cellulose polymers such as methyl cellulose, nitro cellulose, ethyl cellulose, hydroxypropyl methylcellulose, hydroxyethyl cellulose, sodium cellulose sulfate, hydroxypropylcellulose, sodium carboxymethyl cellulose (CMC), microcrystalline cellulose, and cellulose powder; alginate polymers such as sodium alginate and propyleneglycol alginate;

synthetic water-soluble polymers including vinyl polymers such as polyvinyl alcohol, polyvinyl methyl ether, polyvinylpyrrolidone, carboxylvinyl polymer (Carbopol), and alkyl-modified carboxylvinyl polymers; polyoxyethylene polymers such as polyethylene glycols 2000, 4000, and 6000; polyoxyethylene polyoxypropylene copolymers; acrylic polymers such as sodium polyacrylate, polyethyl acrylate, and polyacrylamide; polyethyleneimine; and cationic polymers;

inorganic water-soluble polymers such as bentonite, aluminum magnesium silicate (bee gum), Laponite, hectorite, and silicic anhydride;

thickeners such as casein, dextrin, gelatine, sodium pectate, methylcellulose, CMC, hydroxyethylcellulose, hydroxypropylcellulose, PVA, PVM, PVP, sodium polyacrylate, carboxylvinyl polymer, locust been gum, guar gum, tamarind gum, dimethyl dialkyl ammonium cellulose sulfate, xanthane gum, aluminum magnesium silicate, bentonite, and hectorite;

powder ingredients including inorganic powders such as sericite, muscovite, golden mica, synthetic mica, lepidolite, biotite, vermiculite, magnesium carbonate, calcium carbonate, aluminum silicate, barium silicate, calcium silicate, magnesium silicate, strontium silicate, tungstic acid metal salt, magnesium, silica, zeolite, barium sulfate, calcined calcium sulfate (calcined gypsum), calcium phosphate, fluorapatite, hydroxyapatite, ceramic powder, metallic soap (such as calcium palmitate and aluminum stearate), and boron nitride; and ogranic powders such as polyamide resin powder (nylon powder), polyethylene powder, polymethyl methacrylate powder, polystyrene powder, styrene-acrylic acid copolymer resin powder, benzoguanamine resin powder, poly(tetrafluoroethylene) powder, and cellulose powder;

inorganic white pigments such as titanium dioxide and zinc oxide; inorganic red pigments such as red iron oxide and iron titanate; inorganic brown pigments such as $\gamma$-iron oxide; inorganic yellow pigments such as yellow iron oxide and loess; inorganic black pigments such as black iron oxide, carbon black, and lower titanium oxide; inorganic violet pigments such as manganese violet and cobalt violet; inorganic green pigments such as chromium oxide, chromium hydroxide, and cobalt titanate; inorganic blue pibments such as ultramarine and prussian blue; pearl pigments such as titanium-oxide-coated mica, titanium-oxide-coated bismuth oxychloride, titanium-oxide-coated talc, colored-titanium-oxide-coated mica, bismuth oxychloride, and fish scale flake; metallic powder pigments such as aluminum powder and copper powder; organic pigments such as Red #201, Red #202, Red #204, Red #205, Red #220, Red #226, Red #228, Red #405, Orange #203, Orange #204, Yellow #205, Yellow #401, and Blue #404; zirconium-, barium-, or aluminum-lake-organic pigments such as Red #3, Red #104, Red #106, Red #227, Red #230, Red #401, Red #505, Orange #205, Yellow #4, Yellow #5, Yellow #202, Yellow #203, Green #3, and Blue #1; natural colors such as chlorophyll and $\beta$-carotene; coloring agents such as titanium yellow, carthamin, and safflower red; perfumes; water; and alcohols.

Specific formulations of the external use compositions of the present invention are described below in the Examples hereunder.

Examples

The present invention will next be described in more detail by way of Examples of the external use compositions A and B, which should not be construed as limiting the invention thereto. Unless otherwise specified, the amount of an incorporated ingredient represents weight % with respect to the total of the composition containing the ingredient.

I. Examples of external use composition A

[Test Example A]

In order to evaluate the intended effects of the external use composition A, the below-described tests were performed.

1A. UV-shielding test (sunburn-cell-production inhibition test)

A mixture of a thiol compound and/or a zinc compound was applied onto the back of healthy men, and the mixture was evaluated in terms of the effect of preventing production of sunburn cells (epidermal cells damaged by sunburn: which, when subjected to hematoxylin-eosin staining, are verified as cells with uniformly eosinophilic cytoplasm and a vacuolated, dense nucleus clearly stained with hematoxylin).

Specifically, the below-described "W/O emulsion composition containing a thiol compound and/or a metallic compound," which had been prepared through a conventional method in advance, was applied onto the back of each man for three days. Twenty-four hours after final application of the W/O emulsion composition, his back was irradiated with UV rays (UVB + UVA) of $2 \times 10^5$ J/m$^2$. Twenty-four hours after irradiation of the UV rays, a piece of skin specimen was collected from his back, a tissue sample was prepared therefrom, and the sample was subjected to hematoxylin-eosin staining.

Sunburn cells in the thus-prepared stained tissue sample were counted, and the effect of the composition for preventing production of sunburn cells was evaluated on the basis of the number of sunburn cells. The sunburn-cell-production inhibitory action was graded according to the below-described four ratings.

The results are shown in Table 1A.

| Formulation of W/O emulsion composition containing a thiol compound and/or a metallic compound | |
|---|---|
| Glyceryl monostearate | 0.5 wt.% |

(continued)

| Formulation of W/O emulsion composition containing a thiol compound and/or a metallic compound | |
| --- | --- |
| Isostearic acid | 3.0 |
| Liquid paraffin | 15.0 |
| Thiol compound (shown in Table 1A) | 1.0 |
| Metallic compound (shown in Table 1A) | 1.0 |
| Purified water | 79.5 |

| Evaluation ratings | |
| --- | --- |
| | The number of sunburn cells per $mm^2$ (X) |
| D: many sunburn cells were observed | $2 < X$ |
| C: some sunburn cells were observed | $1 < X \leq 2$ |
| B: a few sunburn cells were observed | $0 < X \leq 1$ |
| A: no sunburn cells were observed | 0 |

Table 1A

| Thiol compound | Metallic salt | Evaluation results |
| --- | --- | --- |
| None | None | D |
| None | Aluminum oxide | D |
| None | Magnesium chloride | D |
| None | Iron(II) sulfate | D |
| None | Titanium oxide | D |
| None | Zinc gluconate | C |
| None | Basic zinc carbonate | C |
| None | Zinc stearate | C |
| None | Zinc undecylenate | C |
| None | Zinc oxide | C |
| None | Zinc myristate | C |
| None | Zinc laurate | C |
| Glutathione | None | C |
| N-Acetylcysteine | None | C |
| Glutathione | Zinc gluconate | A |
| Glutathione | Basic zinc carbonate | A |
| Glutathione | Zinc stearate | A |
| Glutathione | Zinc undecylenate | A |
| Glutathione | Zinc oxide | A |
| Glutathione | Zinc myristate | A |
| N-Acetylcysteine | Zinc laurate | A |
| N-Acetylcysteine | Zinc gluconate | A |

As is apparent from Table 1A, a thiol compound such as glutathione or N-acetylcysteine exhibits the effect of preventing production of sunburn cells, and a zinc compound exhibits the effect of preventing production of sunburn cells to some extent.

The results show that, when a zinc compound and a thiol compound are incorporated in combination into the composition, surprisingly, the effect of the composition for preventing production of sunburn cells; i.e., UV-shielding effect, is drastically enhanced.

2A. Thiol compound stability test

In order to evaluate stability of glutathione (a thiol compound), powder of a metallic salt or a metallic oxide, and glutathione were incorporated in combination into an aqueous solution, and change in the odor of the solution was evaluated.

Specifically, an aqueous solution containing powder of a metallic compound shown in Table 2A and glutathione (the formulation of the solution is described below) was allowed to stand at 50°C for two weeks. Thereafter, change in the odor of the solution was organoleptically evaluated by three expert panelists on the basis of the following ratings. The results are shown in Table 2A.

Evaluation ratings

A:   No offensive odor

B:   Little offensive odor

C:   Some offensive odor

D:   Offensive odor

| Formulation of an aqueous solution containing a metallic salt compound | |
|---|---|
| Purified water | 98.0 wt.% |
| Glutathione | 1.0 |
| Metallic salt compound (shown in Table 2A) | 1.0 |

Table 2A

| Metallic powder | Evaluation results |
|---|---|
| Control (purified water 99%, glutathione 1%) | D |
| Aluminum oxide | D |
| Aluminum sulfate | D |
| Magnesium chloride | D |
| Magnesium sulfate | D |
| Magnesium nitrate | D |
| Iron(II) sulfate | D |
| Titanium oxide | D |
| Alumina-treated titanium oxide | D |
| Zinc sulfate | A |
| Zinc carbonate | B |
| Zinc gluconate | A |
| Basic zinc carbonate | A |
| Zinc chloride | A |

Table 2A   (continued)

| Metallic powder | Evaluation results |
|---|---|
| Zinc laurate | B |
| Zinc palmitate | B |
| Zinc para-phenolsulfonate | A |
| Zinc undecylenate | A |
| Zinc stearate | B |

As is apparent from Table 2A, an aqueous solution containing glutathione and powder of a zinc salt compound does not generate an offensive odor; i.e., glutathione is stable in the solution.

Therefore, when glutathione and a zinc salt compound are incorporated in combination into an external use composition, the resultant composition is stable and does not generate an offensive odor.

Milky lotions were prepared according to the formulations shown in Tables 3A, 4A, and 5A, and each of the emulsions was subjected to the aforementioned "thiol compound stability test." The results are shown in Tables 3A, 4A, and 5A.

Table 3A

|  | Examples | | | | | Comp. Ex.1A |
|---|---|---|---|---|---|---|
|  | 1A | 2A | 3A | 4A | 5A |  |
| Liquid paraffin | 30.0 | 30.0 | 30.0 | 30.0 | 30.0 | 30.0 |
| Cetyl 2-ethyl hexanoate | 5.0 | 5.0 | 5.0 | 5.0 | 5.0 | 5.0 |
| Jojoba oil | 5.0 | 5.0 | 5.0 | 5.0 | 5.0 | 5.0 |
| Decamethylcyclopenta-Siloxane | 20.0 | 20.0 | 20.0 | 20.0 | 20.0 | 20.0 |
| Polyoxyalkylene-modified organopolysiloxane | 1.0 | 1.0 | 1.0 | 1.0 | 1.0 | 1.0 |
| Zinc gluconate | 5.0 | 5.0 | 5.0 | 5.0 | 5.0 | - |
| Organo modified Bentonite | 1.5 | 1.5 | 1.5 | 1.5 | 1.5 | 1.5 |
| 1,3-Butylene glycol | 5.0 | - | 5.0 | 5.0 | 5.0 | 5.0 |
| EDTA.3Na.2H$_2$O | 0.5 | 0.5 | - | 0.5 | 0.5 | 0.5 |
| Tocopherol acetate | 1.0 | 1.0 | 1.0 | - | 1.0 | 1.0 |
| Methyl paraben | 0.5 | 0.5 | 0.5 | 0.5 | - | 0.5 |
| Glutathione | 1.0 | 1.0 | 1.0 | 1.0 | 1.0 | 1.0 |
| Purified water | Bal. | Bal. | Bal. | Bal. | Bal. | Bal. |
| Evaluation of Odor | A | A | A | A | A | D |
| Bal. = Balance | | | | | | |

Table 4A

|  | Examples | | | | | Comp. Ex. 2A |
|---|---|---|---|---|---|---|
|  | 6A | 7A | 8A | 9A | 10A |  |
| Liquid paraffin | 30.0 | 30.0 | 30.0 | 30.0 | 30.0 | 30.0 |
| Cetyl 2-ethyl hexanoate | 5.0 | 5.0 | 5.0 | 5.0 | 5.0 | 5.0 |
| Jojoba oil | 5.0 | 5.0 | 5.0 | 5.0 | 5.0 | 5.0 |
| Decamethylcyclopenta-Siloxane | 20.0 | 20.0 | 20.0 | 20.0 | 20.0 | 20.0 |
| Polyoxyalkylene-Modified Organopolysiloxane | 1.0 | 1.0 | 1.0 | 1.0 | 1.0 | 1.0 |
| Basic zinc carbonate | 5.0 | 5.0 | 5.0 | 5.0 | 5.0 | - |
| Organo modified Bentonite | 1.5 | 1.5 | 1.5 | 1.5 | 1.5 | 1.5 |
| 1,3-Butylene glycol | 5.0 | - | 5.0 | 5.0 | 5.0 | 5.0 |
| EDTA.3Na.2H$_2$O | 0.5 | 0.5 | - | 0.5 | 0.5 | 0.5 |
| Tocopherol acetate | 1.0 | 1.0 | 1.0 | - | 1.0 | 1.0 |

Table 4A   (continued)

| | Examples | | | | | Comp. Ex. 2A |
|---|---|---|---|---|---|---|
| | 6A | 7A | 8A | 9A | 10A | |
| Methyl paraben | 0.5 | 0.5 | 0.5 | 0.5 | - | 0.5 |
| N-Acetylcysteine | 1.0 | 1.0 | 1.0 | 1.0 | 1.0 | 1.0 |
| Purified water | Bal. | Bal. | Bal. | Bal. | Bal. | Bal. |
| Evaluation of Odor | A | A | A | A | A | D |
| Bal. = Balance | | | | | | |

Table 5A

| | Examples | | | | | Comp. Ex. 3A |
|---|---|---|---|---|---|---|
| | 11A | 12A | 13A | 14A | 15A | |
| Liquid paraffin | 30.0 | 30.0 | 30.0 | 30.0 | 30.0 | 30.0 |
| Cetyl 2-ethyl Cetyl 2-ethyl hexanoate | 5.0 | 5.0 | 5.0 | 5.0 | 5.0 | 5.0 |
| Jojoba oil | 5.0 | 5.0 | 5.0 | 5.0 | 5.0 | 5.0 |
| Decamethylcyclopenta-Siloxane | 20.0 | 20.0 | 20.0 | 20.0 | 20.0 | 20.0 |
| Polyoxyalkylene-Modified Organopolysiloxane | 1.0 | 1.0 | 1.0 | 1.0 | 1.0 | 1.0 |
| Zinc chloride | 5.0 | 5.0 | 5.0 | 5.0 | 5.0 | - |
| Organo modified Bentonite | 1.5 | 1.5 | 1.5 | 1.5 | 1.5 | 1.5 |
| 1,3-Butylene glycol | 5.0 | - | 5.0 | 5.0 | 5.0 | 5.0 |
| EDTA.3Na.2$H_2$O | 0.5 | 0.5 | - | 0.5 | 0.5 | 0.5 |
| Tocopherol acetate | 1.0 | 1.0 | 1.0 | - | 1.0 | 1.0 |
| Methyl paraben | 0.5 | 0.5 | 0.5 | 0.5 | - | 0.5 |
| Glutathione | 5.0 | 5.0 | - | - | - | 5.0 |
| N-Acetylcysteine | - | - | 5.0 | 5.0 | 5.0 | - |
| Antioxidant(1) | - | 1.0 | - | - | - | 1.0 |
| Antioxidant(2) | - | - | 1.0 | - | 1.0 | - |
| Purified water | Bal. | Bal. | Bal. | Bal. | Bal. | Bal. |
| Evaluation of Odor | B | A | A | B | A | D |
| Bal. = Balance  Antioxidant(1): dibutylhydroxytoluene  Antioxidant(2): butylhydroxyanisole | | | | | | |

As is apparent from the results shown in Tables 3A through 5A, even when the external use composition of each Example containing a thiol compound and a zinc compound in combination is allowed to stand under stringent conditions for a long period of time, the composition does not generate an offensive odor attributed to modification of the thiol compound, and the composition is very stable. In the case in which an antioxidant is added to the external use composition, even when a large amount of a thiol compound is incorporated into the composition, the composition is very stable.

Typical formulation examples of the external use composition A are described below. Even when the composition of each formulation was allowed to stand at 50°C for two months, a thiol compound contained in the composition was stable, and the composition did not generate an offensive odor attributed to modification of the incorporated thiol compound. Even when the external use composition A assumes an arbitrary product form, the stability of a thiol compound in the composition is drastically enhanced; i.e., the composition fully exerts one of the intended effects.

When the composition of each formulation was subjected to the aforementioned UV-shielding test, the composition exerted the effect of preventing production of sunburn cells. Briefly, when a thiol compound and a zinc compound are incorporated in combination into the composition, the composition exerts a remarkably excellent UV-shielding effect.

[Example 16A]      Powder-containing gel

A gel having the following formulation was prepared by dissolving the aqueous phase ingredients while stirring and adding thereto the alcohol phase ingredients while stirring.

| Ingredient | Amount (wt.%) |
|---|---|
| A. Aqueous phase | |
| Purified water | balance |
| Polyethylene glycol 400 | 5.0 |
| Propylene glycol | 5.0 |
| Dipotassium glycyrrhizinate | 0.1 |
| Sodium hyaluronate | 0.05 |
| Carboxyvinyl polymer | 0.5 |
| Potassium hydroxide | 0.2 |
| Sodium hydroxymethoxybenzophenone sulfonate | 0.1 |
| Glutathione | 0.5 |
| Zinc gluconate | 0.5 |
| B. Alcohol phase | |
| Ethanol | 10.0 |
| POE(25) octyldodecyl ether | 0.5 |
| 2-Ethylhexyl p-methoxycinnamate | 0.05 |
| Tocopheryl acetate | 0.1 |
| Methyl paraben | 0.1 |
| Perfume | suitable amount |

[Example 17A]      Milky lotion (1)

A milky lotion having the following formulation was prepared by adding the oily phase ingredients to the aqueous phase ingredients and emulsifying the resultant mixture in an emulsifier.

| Ingredient | Amount (wt.%) |
|---|---|
| A. Oily phase | |
| Stearic acid | 2.0 |
| Cetanol | 1.0 |
| Vaseline | 2.0 |
| Liquid paraffin | 9.0 |
| Cetyl 2-ethylhexanoate | 1.0 |
| Jojoba oil | 1.0 |
| Squalane | 2.0 |
| Pentaerythritol tetra-2-ethylhexanoate | 3.0 |
| Methylphenyl polysiloxane | 2.0 |
| Evening primrose oil | 0.5 |
| Glyceryl octanoate di-p-methoxycinnamate | 1.5 |
| POE(10) monooleate | 0.1 |
| Butyl paraben | 0.2 |
| Perfume | suitable amount |
| B. Aqueous phase | |
| Propylene glycol | 5.0 |
| 1,3-Butylene glycol | 2.0 |

(continued)

| Ingredient | Amount (wt.%) |
|---|---|
| B. Aqueous phase | |
| Arbutin | 5.0 |
| Carboxyvinyl polymer | 0.2 |
| Triethanolamine | 0.2 |
| N-Acetylcysteine | 3.0 |
| Zinc sulfate | 1.0 |
| Sodium bisulfite | 0.5 |
| Purified water | balance |

[Example 18A]        Milky lotion (2)

In a manner similar to that employed in Example 17A, a milky lotion having the following formulation was prepared.

| Ingredient | Amount (wt.%) |
|---|---|
| A. Oily phase | |
| Stearic acid | 2.0 |
| Cetanol | 1.0 |
| Vaseline | 2.0 |
| Liquid paraffin | 9.0 |
| Cetyl 2-ethylhexanoate | 1.0 |
| Jojoba oil | 1.0 |
| Squalane | 2.0 |
| Pentaerythritol | |
| tetra-2-ethylhexanoate | 3.0 |
| Methylphenylpolysiloxane | 2.0 |
| Evening primrose oil | 0.5 |
| p-Aminobenzoic acid | 1.5 |
| POE(10) monooleate | 0.1 |
| Stearic-acid-treated zinc oxide microparticles | 5.0 |
| (Average particle size: 0.02 µm) Butyl paraben | 0.2 |
| Perfume | suitable amount |
| B. Aqueous phase | |
| Propylene glycol | 5.0 |
| 1,3-Butylene glycol | 2.0 |
| Arbutin | 5.0 |
| Carboxyvinyl polymer | 0.2 |
| Triethanolamine | 0.2 |
| N-Acetylcysteine | 3.0 |
| Zinc white | 5.0 |
| Purified water | balance |

[Example 19A]        Cream (1)

A cream having the following formulation was prepared by adding the oily phase ingredients to the aqueous phase ingredients and emulsifying the resultant mixture in an emulsifier.

| Ingredient | Amount (wt.%) |
|---|---|
| A. Oily phase | |
| Stearic acid | 2.0 |
| Stearyl alcohol | 7.0 |
| Hydrogenated lanolin | 2.0 |
| Olive oil | 1.0 |
| Glyceryl tri-2-ethylhexanoate | 3.0 |
| Octyldodecanol | 5.0 |
| POE(25) cetyl ether | 3.0 |
| Glyceryl monostearate | 2.0 |
| Octyl salicylate | 0.5 |
| 2-Hydroxy-4-methoxybenzophenone | 0.5 |
| Basic zinc carbonate | 5.0 |
| Propyl paraben | 0.3 |
| Perfume | suitable amount |
| B. Aqueous phase | |
| 1,3-Butylene glycol | 6.0 |
| Dipropylene glycol | 3.0 |
| Glycerin | 4.0 |
| Tranexamic acid | 2.0 |
| Magnesium L-ascorbyl-2-phosphate | 0.1 |
| Pantothenic acid | 0.1 |
| Glutathione | 2.0 |
| Purified water | balance |

[Example 20A]      Cream (2)

In a manner similar to that employed in Example 19A, a cream having the following formulation was prepared.

| Ingredient | Amount (wt.%) |
|---|---|
| A. Oily phase | |
| Stearic acid | 2.0 |
| Stearyl alcohol | 7.0 |
| Hydrogenated lanolin | 2.0 |
| Olive oil | 1.0 |
| Glyceryl tri-2-ethylhexanoate | 3.0 |
| Octyldodecanol | 5.0 |
| POE(25) Cetyl ether | 3.0 |
| Glyceryl monostearate | 2.0 |
| 2-Ethylhexyl p-dimethylaminobenzoate | 0.5 |
| Dextrin-palmitate-treated zinc oxide (Average particle size: 0.04 μm) | 5.0 |
| Zinc laurate | 2.5 |
| Propyl paraben | 0.3 |
| Perfume | suitable amount |
| B. Aqueous phase | |
| 1,3-Butylene glycol | 6.0 |
| Dipropylene glycol | 3.0 |
| Glycerin | 4.0 |

(continued)

| Ingredient | Amount (wt.%) |
|---|---|
| B. Aqueous phase | |
| Tranexamic acid | 2.0 |
| Magnesium L-ascorbyl-2-phosphate | 0.1 |
| Pantothenic acid | 0.1 |
| Glutathione | 2.0 |
| Purified water | balance |

[Example 21A]    Two-layer lotion (1)

A two-layer lotion having the following formulation was prepared by dispersing the aqueous phase ingredients while stirring and adding thereto the alcohol phase ingredients.

| Ingredient | Amount (wt.%) |
|---|---|
| A. Aqueous phase | |
| Purified water | balance |
| Propylene glycol | 4.0 |
| Allantoin | 0.2 |
| Sodium chloride | 0.1 |
| Bentonite | 1.0 |
| Talc | 0.5 |
| Cellulose powder | 0.5 |
| Silica | 1.0 |
| Sodium hydroxymethoxybenzophenone sulfonate | 0.1 |
| Glutathione | 0.5 |
| Basic zinc carbonate | 1.0 |
| B. Alcohol phase | |
| Ethanol | 15.0 |
| Methyl paraben | 0.1 |
| Menthol | 0.05 |
| POE(60) glyceryl monoisostearate | 0.5 |
| Tocopherol acetate | 0.01 |
| Perfume | suitable amount |

[Example 22A]    Two-layer lotion (2)

In a manner similar to that employed in Example 21A, a two-layer lotion having the following formulation was prepared.

| Ingredient | Amount (wt.%) |
|---|---|
| A. Aqueous phase | |
| Purified water | balance |
| Propylene glycol | 4.0 |
| Allantoin | 0.2 |
| Sodium chloride | 0.1 |
| Bentonite | 1.0 |
| Talc | 0.5 |
| Cellulose powder | 0.5 |
| Silica | 1.0 |

(continued)

| Ingredient | Amount (wt.%) |
|---|---|
| A. Aqueous phase | |
| Sodium hydroxymethoxybenzophenone sulfonate | 0.1 |
| Glutathione | 0.5 |
| Zinc myristate | 1.5 |
| Zinc oxide microparticles | 1.0 |
| (Average particle size: 0.02 µm) | |
| B. Alcohol phase | |
| Ethanol | 15.0 |
| Methyl paraben | 0.1 |
| Menthol | 0.05 |
| POE(60) glyceryl monoisostearate | 0.5 |
| Tocopherol acetate | 0.01 |
| Perfume | suitable amount |

[Example 23A]    Peel-off pack (1)

A peel-off pack having the following composition was prepared by dissolving the aqueous phase ingredients while stirring and adding thereto the alcohol phase ingredients.

| Ingredient | Amount (wt.%) |
|---|---|
| A. Aqueous phase | |
| Purified water | balance |
| Polyethylene glycol 1500 | 5.0 |
| Polyvinyl alcohol | 13.0 |
| Placental extract | 0.3 |
| N-Acetylcysteine | 0.5 |
| Zinc citrate | 1.0 |
| Zinc oxide microparticles | 1.0 |
| (Average particle size: 0.1 µm) | |
| B. Alcohol phase | |
| Ethanol | 7.0 |
| POE(20) oleyl alcohol ether | 1.0 |
| Methyl paraben | 0.2 |
| Myristic-acid-treated zinc oxide microparticles | 1.0 |
| Perfume | suitable amount |

[Example 24A]    Peel-off pack (2)

In a manner similar to that employed in Example 23A, a peel-off pack having the following formulation was prepared.

| Ingredient | Amount (wt.%) |
|---|---|
| A. Aqueous phase | |
| Purified water | balance |
| Polyethylene glycol 1500 | 5.0 |
| Polyvinyl alcohol | 13.0 |
| Placental extract | 0.3 |

(continued)

| Ingredient | Amount (wt.%) |
|---|---|
| A. Aqueous phase | |
| N-Acetylcysteine | 0.5 |
| Glutathione | 5.0 |
| Zinc p-phenolsulfonate | 1.0 |
| Zinc pyrithione | 0.5 |
| Zinc stearate | 0.2 |
| Zinc sulfate | 0.2 |
| Zinc white | 1.0 |
| Zinc oxide microparticles | 1.0 |
| (Average particle size: 0.1 μm) | |
| B. Alcohol phase | |
| Ethanol | 7.0 |
| POE(20) oleyl alcohol ether | 1.0 |
| Methyl paraben | 0.2 |
| Myristic-acid-treated zinc oxide | 1.0 |
| Perfume | suitable amount |

[Example 25A]     Sunscreen cream (1)

A sunscreen cream having the following formulation was prepared by adding the oily phase ingredients to the aqueous phase ingredients while emulsifying the resultant mixture in an emulsifier.

| Ingredient | Amount (wt.%) |
|---|---|
| A. Oily phase | |
| Octyl methoxycinnamate | 5.0 |
| Decamethylcyclopentasiloxane | 20.0 |
| Methyl polysiloxane | 5.0 |
| POE glyceryl triisostearate | 1.5 |
| Organo modified clay mineral (Benton 38) | 0.5 |
| Silicone resin | 5.0 |
| Tocopheryl acetate | 0.05 |
| Methyl paraben | 0.5 |
| Alumina-treated titanium dioxide | 5.0 |
| Zinc pyrithione | 1.0 |
| Perfume | suitable amount |
| B. Aqueous phase | |
| 1,3-Butylene glycol | 5.0 |
| Glycerin | 5.0 |
| EDTA · 3Na-2H$_2$O | 0.5 |
| Glutathione | 1.0 |
| Purified water | balance |

[Example 26A]     Sunscreen cream (2)

In a manner similar to that employed in Example 25A, a sunscreen cream having the following formulation was prepared.

| Ingredient | Amount (wt.%) |
|---|---|
| A. Oily phase | |
| Octyl methoxycinnamate | 5.0 |
| Decamethylcyclopentasiloxane | 20.0 |
| Methyl polysiloxane | 5.0 |
| POE glyceryl triisostearate | 1.5 |
| Organo modified clay mineral (Benton 38) | 0.5 |
| Silicone resin | 5.0 |
| Tocopheryl acetate | 0.05 |
| Methyl paraben | 0.5 |
| Alumina-treated titanium dioxide | 5.0 |
| Silicone-treated zinc oxide | 5.0 |
| (Average particle size: 0.02 μm; the silicone treatment employed herein refers to the method disclosed in Japanese Patent Publication (kokoku) No. 1-54381) | |
| Zinc undecylenate | 0.3 |
| Perfume | suitable amount |
| B. Aqueous phase | |
| 1,3-Butylene glycol | 5.0 |
| Glycerin | 5.0 |
| EDTA · 3Na-2H$_2$O | 0.5 |
| Glutathione | 1.0 |
| Purified water | balance |

I. Examples of external use composition B

[Test Example B]

1B. Metallothionein induction test

A polar oil derivative (having an I.O.B. value of 0.1 or more), a higher fatty acid zinc salt, or a mixture thereof was applied onto the back of healthy men, and the compound was evaluated in terms of the effect of inducing production of metallothionein.

Specifically, the below-described "W/O emulsion composition containing a polar oil derivative and/or a higher fatty acid zinc salt," which had been prepared through a conventional method in advance, was applied onto the back of each man. Fifteen hours after application of the "W/O emulsion composition containing a methoxycinnamic acid derivative and/or a higher fatty acid zinc salt," a piece of skin specimen (biopsy sample) was collected from his back, a tissue sample was prepared therefrom, and the sample was subjected to immunohistochemical staining by use of an anti horse liver metallothionein antibody (product of DAKO).

Staining of the thus-prepared sample with respect to the metallothionein antibody was evaluated on the basis of the following four ratings.

The results are shown in Table 1B.

| Formulation of W/O emulsion composition containing a polar oil derivative and a higher fatty acid zinc salt | |
|---|---|
| Glyceryl monostearate | 0.5 wt.% |
| Isostearic acid | 3.0 wt.% |
| Liquid paraffin | 15.0 wt.% |
| Polar oil derivative (shown in Table 1B) | 1.0 wt.% |
| Higher fatty acid zinc salt (shown in Table 1B) zz | 1.0 wt.% |
| Purified water | 79.5 wt.% |

Evaluation ratings

D: many sunburn cells were observed
C: some sunburn cells were observed
B: a few sunburn cells were observed
A: no sunburn cells were observed

Table 1B

| Polar oil derivative | Higher fatty acid zinc salt | Evaluation results |
|---|---|---|
| None | None | D |
| 2-Ethylhexyl p-methoxycinnamate (Octyl p-methoxycinnamate) | None | D |
| None | Zinc laurate | D |
| 2-Ethylhexyl p-methoxycinnamate (Octyl p-methoxycinnamate) | Zinc laurate | A |
| None | Zinc myristate | D |
| 2-Ethylhexyl p-methoxycinnamate (Octyl p-methoxycinnamate) | Zinc myristate | A |
| Ethyl p-methoxycinnamate | None | D |
| None | Zinc stearate | D |
| Ethyl p-methoxycinnamate | Zinc stearate | A |
| Isopropyl p-methoxycinnamate | None | D |
| None | Zinc undecylenate | D |
| Isopropyl p-methoxycinnamate | Zinc undecylenate | A |
| None | Zinc oleate | D |
| Isopropyl p-methoxycinnamate | Zinc oleate | C |
| Di-2-ethylhexylsuccinate | None | D |
| Di-2-ethylhexylsuccinate | Zinc laurate | B |
| Glyceryl tri-2-ethylhexanoate | None | D |
| Glyceryl tri-2-ethylhexanoate | Zinc myristate | B |
| Pentaerythritol tetra-2-ethylhexanoate | None | D |
| Pentaerythritol tetra-2-ethylhexanoate | Zinc stearate Zinc stearate | A |
| Cetyl octanoate | None | D |
| Cetyl octanoate | Zinc undecylenate | B |

As is apparent from Table 1B, when a higher fatty acid zinc salt or a polar oil derivative (having an I.O.B. value of 0.1 or more) is singly incorporated into the composition, and the composition is administered transdermally, the composition does not exert the effect to induce production of metallothionein in skin cells. In contrast, when a higher fatty acid zinc salt and a polar oil derivative are incorporated in combination into the composition, and the composition is administered transdermally, the composition exerts a drastically enhanced effect of inducing production of metallothionein.

The results show that, when a higher fatty acid zinc salt is dissolved in a polar oil derivative, and as a result, the zinc salt is absorbed transdermally, and the zinc salt induces production of metallothionein in skin cells.

2B. Sunburn-cell-production inhibition test

A polar oil derivative, a higher fatty acid zinc salt, or a mixture thereof was applied onto the back of healthy men, and then the compound was evaluated in terms of the effect of preventing production of sunburn cells.

Specifically, the below-described "W/O emulsion composition containing a polar oil derivative and/or a higher fatty acid zinc salt," which had been prepared through a conventional method in advance, was applied onto the back of each man for three days. Twenty-four hours after final application of the "W/O emulsion composition containing a methoxycinnamic acid derivative and/or a higher fatty acid zinc salt," the composition was wiped off the surface of the back skin, and then his back was irradiated with UV rays (UVB + UVA) of $2 \times 10^5$ J/m$^2$. Twenty-four hours after irradiation with the UV rays, a piece of skin specimen (biopsy sample) was collected from his back, a tissue sample was prepared therefrom, and the sample was subjected to hematoxylin-eosin staining.

Sunburn cells in the thus-prepared stained tissue sample were counted, and the effect of the composition for preventing production of sunburn cells was evaluated on the basis of the number of sunburn cells. The sunburn-cell-production inhibitory action was graded according to the below-described four ratings.

The results are shown in Table 2B.

| Formulation of W/O emulsion composition containing a polar oil derivative and a higher fatty acid zinc salt | |
|---|---|
| Glyceryl monostearate | 0.5 wt.% |
| Isostearic acid | 3.0 wt.% |
| Liquid paraffin | 15.0 wt.% |
| Polar oil derivative (shown in Table 2B) | 1.0 wt.% |
| Higher fatty acid zinc salt (shown in Table 2B) | 1.0 wt.% |
| Purified water | 79.5 wt.% |

| Evaluation ratings | | |
|---|---|---|
| | | The number (X) of sunburn cells per mm$^2$ |
| D | many sunburn cells were observed | $2 < X$ |
| C | some sunburn cells were observed | $1 < X \leq 2$ |
| B | a few sunburn cells were observed | $0 < X \leq 1$ |
| A | no sunburn cells were observed | 0 |

Table 2B

| Polar oil derivative | Higher fatty acid zinc salt | Evaluation results |
|---|---|---|
| None | None | D |
| 2-Ethylhexyl p-methoxycinnamate (Octyl p-methoxycinnamate) | None | C |
| None | Zinc laurate | D |
| 2-Ethylhexyl p-methoxycinnamate (Octyl p-methoxycinnamate) | Zinc laurate | A |
| None | Zinc myristate | D |
| 2-Ethylhexyl p-methoxycinnamate (Octyl p-methoxycinnamate) | Zinc myristate | A |
| Ethyl p-methoxycinnamate | None | C |
| None | Zinc stearate | D |
| Ethyl p-methoxycinnamate | Zinc stearate | A |
| Isopropyl p-methoxycinnamate | None | C |
| None | Zinc undecylenate | D |
| Isopropyl p-methoxycinnamate | Zinc undecylenate | A |
| None | Zinc oleate | D |
| Isopropyl p-methoxycinnamate | Zinc oleate | B |
| Di-2-ethylhexylsuccinate | None | D |

Table 2B   (continued)

| Polar oil derivative | Higher fatty acid zinc salt | Evaluation results |
|---|---|---|
| Di-2-ethylhexylsuccinate | Zinc laurate | B |
| Glyceryl tri-2-ethylhexanoate | None | D |
| Glyceryl tri-2-ethylhexanoate | Zinc myristate | A |
| Pentaerythritol tetra-2-ethylhexanoate | None | D |
| Pentaerythritol tetra-2-ethylhexanoate | Zinc stearate Zinc stearate | A |
| Cetyl octanoate | None | D |
| Cetyl octanoate | Zinc undecylenate | B |

As is apparent from Table 2B, when the composition containing only a higher fatty acid zinc salt is administered transdermally, the composition does not exerts the effect of preventing production of sunburn cells, and when the composition containing a methoxycinnamic acid derivative is administered transdermally, the composition exerts the effect of preventing production of sunburn cells to some extent, the prevention effect being attributed to the UV-absorbing effect of the methoxycinnamic acid derivative. In contrast, when a higher fatty acid zinc salt and a polar oil derivative (having an I.O.B. value of 0.1 or more) are incorporated in combination into the composition, and the composition is administered transdermally, surprisingly, the composition exerts a drastically enhanced effect of preventing production of sunburn cells.

The results agree with the fact that, when a higher fatty acid zinc salt and a polar oil derivative are incorporated in combination into the composition, and the composition is administered transdermally, the composition exerts a drastically enhanced effect of inducing production of metallothionein. The results show that a higher fatty acid zinc salt is dissolved in a polar oil derivative, and as a result, the zinc salt is absorbed transdermally, and the zinc salt induces production of metallothionein in skin cells, and therefore production of sunburn cells is prevented, due to the antioxidative action of metallothionein.

The results also show that, when a higher fatty acid zinc salt and a polar oil derivative are incorporated in combination into the composition, and the composition is administered transdermally, production of sunburn cells is prevented, and consequently, skin damage caused by UV rays, such as sunburn, can be prevented.

Typical formulation examples of the external use composition of the present invention are described below as working examples.

The compositions were subjected to the aforementioned metallothionein induction test and sunburn-cell-production inhibition test. In each test, evaluation of the compositions was rated "A" or "B." The odor of the compositions was organoleptically evaluated by three expert panelists. The results show that the compositions do not generate an offensive odor.

Therefore, even when the external use composition of the present invention assumes an arbitrary product form, the composition fully exerts the intended effects.

[Example 1B]        Powder-containing gel

A gel having the following formulation was prepared by dissolving the aqueous phase ingredients while stirring and adding thereto the alcohol phase ingredients while stirring.

| Ingredient | Amount (wt.%) |
|---|---|
| A. Aqueous phase | |
| Purified water | balance |
| Polyethylene glycol 400 | 5.0 |
| Propylene glycol | 5.0 |
| Dipotassium glycyrrhizinate | 0.1 |
| Sodium hyaluronate | 0.05 |
| Glutathione | 2.0 |
| Carboxyvinyl polymer | 0.5 |
| Potassium hydroxide | 0.2 |

(continued)

| Ingredient | Amount (wt.%) |
|---|---|
| A. Aqueous phase | |
| Sodium hydroxymethoxybenzophenone sulfonate | 0.1 |
| B. Alcohol phase | |
| Ethanol | 10.0 |
| Zinc laurate | 2.0 |
| Methyl octylcinnamate | 10.0 |
| POE(25) 2-octyldodecyl ether | 0.5 |
| 2-Ethylhexyl p-methoxycinnamate (I.O.B. = 0.35) | 0.05 |
| Tocopheryl acetate | 0.1 |
| Methyl paraben | 0.1 |
| Perfume | suitable amount |

[Example 2B]　　　Milky lotion

A milky lotion having the following formulation was prepared by adding the oily phase ingredients to the aqueous phase ingredients and emulsifying the resultant mixture in an emulsifier.

| Ingredient | Amount (wt.%) |
|---|---|
| A. Oily phase | |
| Stearic acid | 2.0 |
| Cetanol | 1.0 |
| Vaseline | 2.0 |
| Liquid paraffin | 9.0 |
| Cetyl 2-ethylhexanoate | 1.0 |
| Jojoba oil | 1.0 |
| Squalane | 2.0 |
| Pentaerythritol tetra-2-ethylhexanoate | 3.0 |
| Methylphenylpolysiloxane | 2.0 |
| Zinc laurate | 1.0 |
| Methyl octylcinnamate | 10.0 |
| Evening primrose oil | 0.5 |
| Glyceryl octanoate di-p-methoxycinnamate (I.O.B. = 0.52) | 1.5 |
| POE(10) monooleate | 0.1 |
| Stearic-acid-treated zinc oxide microparticles | 5.0 |
| 　　　(Average particle size: 0.02 μm) | |
| Butyl paraben | 0.2 |
| Perfume | suitable amount |
| B. Aqueous phase | |
| Propylene glycol | 5.0 |
| 1,3-Butylene glycol | 2.0 |
| Arbutin | 5.0 |
| Carboxyvinyl polymer | 0.2 |
| Triethanolamine | 0.2 |
| N-Acetylcysteine | 3.0 |
| Zinc white | 5.0 |
| Purified water | balance |

[Example 3B]    Cream

A cream having the following formulation was prepared by adding the oily phase ingredients to the aqueous phase ingredients and emulsifying the resultant mixture in an emulsifier.

| Ingredient | Amount (wt.%) |
|---|---|
| A. Oily phase | |
| Stearic acid | 2.0 |
| Stearyl alcohol | 7.0 |
| Hydrogenated lanolin | 2.0 |
| Olive oil | 1.0 |
| Glyceryl tri-2-ethylhexanoate | 3.0 |
| Octyldodecanol | 5.0 |
| POE(25) Cetyl ether | 3.0 |
| Glyceryl monostearate | 2.0 |
| 2-Ethylhexyl p-dimethylaminobenzoate (I.O.B. = 0.35) | 0.5 |
| Dextrin-palmitate-treated zinc oxide | 5.0 |
| (Average particle size: 0.04 µm) | |
| Zinc myristate | 2.0 |
| 2-Ethylhexyl p-methoxycinnamate | 2.0 |
| Methyl octylcinnamate | 10.0 |
| Propyl paraben | 0.3 |
| Perfume | suitable amount |
| B. Aqueous phase | |
| 1,3-Butylene glycol | 6.0 |
| Dipropylene glycol | 3.0 |
| Glycerin | 4.0 |
| Tranexamic acid | 2.0 |
| Magnesium L-ascorbyl-2-phosphate | 0.1 |
| Pantothenic acid | 0.1 |
| Glutathione | 2.0 |
| Purified water | balance |

[Example 4B]    Two-layer lotion

A two-layer lotion having the following formulation was prepared by dispersing the aqueous phase ingredients while stirring and adding thereto the alcohol phase ingredients.

| Ingredient | Amount (wt.%) |
|---|---|
| A. Aqueous phase | |
| Purified water | balance |
| Propylene glycol | 4.0 |
| Allantoin | 0.2 |
| Sodium chloride | 0.1 |
| Bentonite | 1.0 |
| Talc | 0.5 |
| Cellulose powder | 0.5 |
| Silica | 1.0 |
| Sodium hydroxymethoxy-Benzophenone sulfonate | 0.1 |
| Zinc oxide microparticles | 1.0 |
| (Average particle size: 0.02 µm) | |

(continued)

| Ingredient | Amount (wt.%) |
|---|---|
| B. Alcohol phase | |
| Ethanol | 15.0 |
| Zinc stearate | 2.0 |
| Isopropyl p-methoxycinnamate (I.O.B. = 0.49) | 3.0 |
| Ethyl octylcinnamate | 8.0 |
| Methyl paraben | 0.1 |
| Menthol | 0.05 |
| POE(60) glyceryl monoisostearate | 0.5 |
| Tocopheryl acetate | 0.01 |
| Perfume | suitable amount |

[Example 5B]     Peel-off pack

A peel-off pack having the following formulation was prepared by dissolving the aqueous phase ingredients while stirring and adding thereto the alcohol phase ingredients.

| Ingredient | Amount (wt.%) |
|---|---|
| A. Aqueous phase | |
| Purified water | balance |
| Polyethylene glycol 1500 | 5.0 |
| Polyvinyl alcohol | 13.0 |
| Placental extract | 0.3 |
| N-Acetylcysteine | 0.5 |
| Glutathione | 5.0 |
| Zinc white | 1.0 |
| Zinc oxide microparticles (Average particle size: 0.1 μm) | 1.0 |
| B. Alcohol phase | |
| Ethanol | 7.0 |
| POE(20) oleyl alcohol ether | 1.0 |
| Zinc undecylenate | 0.5 |
| Octyl p-methoxycinnamate (I.O.B. = 0.35) | 5.0 |
| Methyl paraben | 0.2 |
| Perfume | suitable amount |

[Example 6B]     Sun-screening cream

A sun-screening cream having the following formulation was prepared by adding the oily phase ingredients to the aqueous phase ingredients while emulsifying the resultant mixture in an emulsifier.

| Ingredient | Amount (wt.%) |
|---|---|
| A. Oily phase | |
| Zinc laurate | 1.0 |
| Zinc undecylenate | 0.3 |
| Methyl octylcinnamate | 10.0 |
| Sodium p-methoxycinnamate (I.O.B. $\geq$ 3.11) | 1.0 |
| Decamethylcyclopentasiloxane | 20.0 |

(continued)

| Ingredient | Amount (wt.%) |
|---|---|
| A. Oily phase | |
| Methyl polysiloxane | 5.0 |
| POE glyceryl triisostearate ester | 1.5 |
| Organo modified clay mineral (Benton 38) | 0.5 |
| Silicone resin | 5.0 |
| Tocopheryl acetate | 0.05 |
| Methyl paraben | 0.5 |
| Alumina-treated titanium oxide | 5.0 |
| Silicone-treated zinc oxide | 5.0 |
| (Average particle size: 0.02 μm; the silicone treatment employed herein refers to the method disclosed in Japanese Patent Publication (*kokoku*) No. 1-54381) | |
| Perfume | suitable amount |
| B. Aqueous phase | |
| 1,3-Butylene glycol | 5.0 |
| Glycerin | 5.0 |
| EDTA · 3Na-2H$_2$O | 0.5 |
| Glutathione | 1.0 |
| Purified water | balance |

[Example 7B]    Powder-containing gel

A gel having the following formulation was prepared by dissolving the aqueous phase ingredients while stirring and adding thereto the alcohol phase ingredients while stirring.

| Ingredient | Amount (wt.%) |
|---|---|
| A. Aqueous phase | |
| Purified water | balance |
| Polyethylene glycol 400 | 5.0 |
| Propylene glycol | 5.0 |
| Dipotassium glycyrrhizinate | 0.1 |
| Sodium hyaluronate | 0.05 |
| Glutathione | 2.0 |
| Carboxyvinyl polymer | 0.5 |
| Potassium hydroxide | 0.2 |
| Sodium hydroxymethoxybenzophenone sulfonate | 0.1 |
| B. Alcohol phase | |
| Ethanol | 10.0 |
| Zinc laurate | 2.0 |
| Methyl octylcinnamate | 10.0 |
| POE(25) 2-octyldodecyl ether | 0.5 |
| Di-2-ethylhexyl succinate (I.O.B. = 0.32) | 0.05 |
| Tocopheryl acetate | 0.1 |
| Methyl paraben | 0.1 |
| Perfume | suitable amount |

[Example 8B]    Milky lotion

A milky lotion having the following formulation was prepared by adding the oily phase ingredients to the aque-

28

ous phase ingredients and emulsifying the resultant mixture in an emulsifier.

| Ingredient | Amount (wt.%) |
|---|---|
| A. Oily phase | |
| Stearic acid | 2.0 |
| Cetanol | 1.0 |
| Vaseline | 2.0 |
| Liquid paraffin | 9.0 |
| Cetyl 2-ethylhexanoate | 1.0 |
| Jojoba oil | 1.0 |
| Squalane | 2.0 |
| Pentaerythritol tetra-2-ethylhexanoate | 3.0 |
| Methylphenylpolysiloxane | 2.0 |
| Zinc laurate | 1.0 |
| Pentaerythritol tetra-2-ethylhexanoate (I.O.B. = 0.35) | 10.0 |
| Evening primrose oil | 0.5 |
| Glyceryl octanoate di-p-methoxy cinnamate | 1.5 |
| POE(10) monooleate | 0.1 |
| Stearic-acid-treated zinc oxide microparticles (Average particle size: 0.02 μm) | 5.0 |
| Butyl paraben | 0.2 |
| Perfume | suitable amount |
| B. Aqueous phase | |
| Propylene glycol | 5.0 |
| 1,3-Butylene glycol | 2.0 |
| Arbutin | 5.0 |
| Carboxyvinyl polymer | 0.2 |
| Triethanolamine | 0.2 |
| N-Acetylcysteine | 3.0 |
| Zinc white | 5.0 |
| Purified water | balance |

[Example 9B]    Cream

A cream having the following formulation was prepared by adding the oily phase ingredients to the aqueous phase ingredients and emulsifying the resultant mixture in an emulsifier.

| Ingredient | Amount (wt.%) |
|---|---|
| A. Oily phase | |
| Stearic acid | 2.0 |
| Stearyl alcohol | 7.0 |
| Hydrogenated lanolin | 2.0 |
| Olive oil | 1.0 |
| Glyceryl tri-2-ethylhexanoate (I.O.B. = 0.34) | 3.0 |
| Octyldodecanol | 5.0 |
| POE(25) Cetyl ether | 3.0 |
| Glyceryl monostearate | 2.0 |
| 2-Ethylhexyl p-dimethylaminobenzoate | 0.5 |
| Dextrin-palmitate-treated zinc oxide (Average particle size: 0.04 μm) | 5.0 |

(continued)

| Ingredient | Amount (wt.%) |
|---|---|
| A. Oily phase | |
| Zinc myristate | 2.0 |
| 2-Ethylhexyl p-methoxycinnamate | 2.0 |
| Glyceryl tri-2-ethylhexanoate | 10.0 |
| Propyl paraben | 0.3 |
| Perfume | suitable amount |
| B. Aqueous phase | |
| 1,3-Butylene glycol | 6.0 |
| Dipropylene glycol | 3.0 |
| Glycerin | 4.0 |
| Tranexamic acid | 2.0 |
| Magnesium L-ascorbyl-2-phosphate | 0.1 |
| Pantothenic acid | 0.1 |
| Glutathione | 2.0 |
| Purified water | balance |

[Example 10B]        Two-layer lotion

A two-layer lotion having the following formulation was prepared by dispersing the aqueous phase ingredients while stirring and adding thereto the alcohol phase ingredients.

| Ingredient | Amount (wt.%) |
|---|---|
| A. Aqueous phase | |
| Purified water | balance |
| Propylene glycol | 4.0 |
| Allantoin | 0.2 |
| Sodium chloride | 0.1 |
| Bentonite | 1.0 |
| Talc | 0.5 |
| Cellulose powder | 0.5 |
| Silica | 1.0 |
| Sodium hydroxymethoxybenzophenone sulfonate | 0.1 |
| Zinc oxide microparticles | 1.0 |
| (Average particle size: 0.02 μm) | |
| B. Alcohol phase | |
| Ethanol | 15.0 |
| Zinc stearate | 2.0 |
| Isopropyl p-methoxycinnamate | 3.0 |
| (I.O.B. = 0.49) | |
| Cetyl octanoate | 8.0 |
| Methyl paraben | 0.1 |
| Menthol | 0.05 |
| POE(60) glyceryl monoisostearate | 0.5 |
| Tocopheryl acetate | 0.01 |
| Perfume | suitable amount |

Industrial Applicability

As described above, the external use compositions of the present invention can promote production of metallothionein in skin cells, and thus prevent skin damage caused by solar UV rays, without any problem in terms of odor.

**Claims**

1. A composition for external use comprising a zinc compound.

2. The composition for external use according to claim 1, which optionally comprises a thiol compound.

3. The composition for external use according to claim 2, wherein the thiol compound is gluthathione derivatives or cysteine derivatives.

4. The composition for external use according to claim 2 or 3, wherein the zinc compound is one or more species selected from the group consisting of zinc oxide, zinc gluconate, basic zinc carbonate, zinc chloride, zinc laurate, zinc myristate, zinc palmitate, zinc para-phenolsulfonate, zinc pyrithionate, zinc stearate, zinc sulfate, zinc undecylenate, zinc acetate, zinc rhodinate, zinc ricinoleate, and zinc neodecanoate.

5. The composition for external use according to claim 4, wherein the zinc compound is zinc oxide.

6. The composition for external use according to any one of claims 2 through 5, wherein the amount of the zinc compound contained in the composition is 0.01-20.0 wt.% based upon the total of the composition.

7. The composition for external use according to any one of claims 2 through 6, wherein the amount of the thiol compound contained in the composition is 0.01-20.0 wt.% based upon the total of the composition.

8. The composition for external use according to any one of claims 2 through 7, wherein the thiol compound contained in the composition is stabilized.

9. The composition for external use according to claim 1, which optionally comprises a polar oil derivative having an I.O.B. value of 0.1 or more, wherein the zinc compound is a higher fatty acid zinc salt.

10. The composition for external use according to claim 9, wherein the polar oil derivative having an I.O.B. value of 0.1 or more is an oil ester derivative, an oil carboxyl derivative, or an oil carboxylic acid salt.

11. The composition for external use according to claim 9, wherein the polar oil derivative having an I.O.B. value of 0.1 or more is one or more species selected from the group consisting of a para-methoxycinnamic acid derivative, di-2-ethylhexyl succinate, pentaerythritol tetra-2-ethylhexanoate, glyceryl tri-2-ethylhexanoate, cetyl octanoate, isopropyl myristate, isopropyl palmitate, butyl stearate, hexyl laurate, myristyl myristate, decyl oleate, hexyldecyl dimethyloctanoate, cetyl lactate, myristyl lactate, isocetyl stearate, cholesteryl 12-hydroxystearate, ethylene glycol di-2-ethylhexanoate, neopentyl glycol dicaprate, diisostearyl malate, glyceryl di-2-heptylundecanoate, trimethylolpropane tri-2-ethylhexanoate, trimethylolpropane triisostearate, cetyl 2-ethylhexanoate, 2-ethylhexyl palmitate, glyceryl trimyristate, glyceryl tri-2-heptylundecanoate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, di-2-hexyldecyl adipate, diisopropyl sebacate, ethyl acetate, butyl acetate, amyl acetate, and triethyl citrate.

12. The composition for external use according to claim 9, wherein the polar oil derivative having an I.O.B. value of 0.1 or more is para-methoxycinnamic acid derivatives.

13. The composition for external use according to claim 12, wherein the para-methoxycinnamic acid derivative is 2-ethylhexyl para-methoxycinnamate.

14. The composition for external use according to any one of claims 9 through 13, wherein the higher fatty acid zinc salt is one or more species selected from the group consisting of zinc laurate, zinc myristate, zinc palmitate, zinc stearate, zinc undecylenate, zinc caprate, zinc oleate, zinc rhodinate, zinc ricinoleate, and zinc neodecanoate.

15. The composition for external use according to any one of claims 9 through 14, wherein the amount of the polar oil derivative having an I.O.B. value of 0.1 or more contained in the composition is 0.01-30.0 wt.% based upon the total of the composition.

16. The composition for external use according to any one of claims 9 through 15, wherein the amount of the higher fatty acid zinc salt contained in the composition is 0.01-20.0 wt.% based upon the total of the composition.

17. The composition for external use according to any one of claims 9 through 16, wherein the higher fatty acid zinc salt is dissolved in the polar oil derivative having an I.O.B. value of 0.1 or more.

18. The composition for external use according to any one of claims 1 through 17, which is a composition for preventing skin damage.

19. The composition for external use according to claim 18, which prevents skin damage caused by exposure of the skin to UV rays.

20. The composition for external use according to claim 18, which promotes production of metallothionein.

21. The composition for external use according to claim 18, which prevents production of sunburn cells in skin cells.

22. The composition for external use according to claim 18, which prevents sunburn.

## INTERNATIONAL SEARCH REPORT

| International application No. |
|---|
| PCT/JP00/00401 |

| A. CLASSIFICATION OF SUBJECT MATTER |
|---|
| Int.Cl$^7$ A61K7/42, 7/00 |

According to International Patent Classification (IPC) or to both national classification and IPC

| B. FIELDS SEARCHED |
|---|
| Minimum documentation searched (classification system followed by classification symbols) |
| Int.Cl$^7$ A61K7/42, 7/00 |

Documentation searched other than minimum documentation to the extent that such documents are included in the fields searched

Electronic data base consulted during the international search (name of data base and, where practicable, search terms used)
CAPLUS (STN)

**C. DOCUMENTS CONSIDERED TO BE RELEVANT**

| Category* | Citation of document, with indication, where appropriate, of the relevant passages | Relevant to claim No. |
|---|---|---|
| X | EP, 914815, A1 (SHISEIDO COMPANY LIMITED), 12 May, 1999 (12.05.99), Example 6 & WO, 98/43597, A1 & JP, 10-330244, A | 1,2,4,6-8, 18-22 |
| X | WO, 96/01101, A1 (THE PROCTER & GAMBLE COMPANY), 18 January, 1996 (18.01.96), Example VII & JP, 10-505326, A & EP, 768866, A1 | 1-8,18-22 |
| X | JP, 10-212211, A (Natl Inst. for Res. in Inorg. Mater.), 11 August, 1998 (11.08.98), example 4 (Family: none) | 1,9-22 |
| X | JP, 10-259142, A (Shiseido Company, Limited. ), 29 September, 1998 (29.09.98), example 7 (Family: none) | 1,9-12,14-22 |
| X | JP, 8-268856, A (Shiseido Company, Limited. ), 15 October, 1996 (15.10.96), example 5 (Family: none) | 1,9-12,14-22 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 April, 2000 (25.04.00) | 02 May, 2000 (02.05.00) |

| Name and mailing address of the ISA/ | Authorized officer |
|---|---|
| Japanese Patent Office | |
| Facsimile No. | Telephone No. |

Form PCT/ISA/210 (second sheet) (July 1992)

**INTERNATIONAL SEARCH REPORT**

| International application No. |
| --- |
| PCT/JP00/00401 |

C (Continuation). DOCUMENTS CONSIDERED TO BE RELEVANT

| Category* | Citation of document, with indication, where appropriate, of the relevant passages | Relevant to claim No. |
| --- | --- | --- |
| X | JP, 10-130130, A (Rejino Color Kogyo K.K.), 19 May, 1998 (19.05.98), Par. Nos. [0010], [0018], example; Par. No. [0034]   (Family: none) | 1,9-11,14-22 |

Form PCT/ISA/210 (continuation of second sheet) (July 1992)

## INTERNATIONAL SEARCH REPORT

International application No.

PCT/JP00/00401

**Box I   Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II   Observations where unity of invention is lacking (Continuation of item 2 of first sheet)**

This International Searching Authority found multiple inventions in this international application, as follows:

Since there have been widely known compositions for external use which contain zinc compounds (see, if necessary, JP, 7-97309, A), the presence of zinc compounds cannot be considered as a technical feature of the present invention. It is understood that contributions of the claimed inventions of this international application to the prior art involve an improvement in the stability of thiol compounds by the combined use of the thiol compounds with zinc compounds as achieved by in the invention as set forth in claim 2 and an improvement in the percutaneous absorbability of higher fatty acid zinc salts by the combined use of the higher fatty acid zinc salts with polar oil derivatives as achieved by the invention as set forth in claim 9. Such being the case, the technical features of these inventions differ from each other and it does not appear that there is a technical relationship between these inventions involving one or more of the same or corresponding technical features. Thus, it is recognized that the international application have two or more inventions.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest.

☐   No protest accompanied the payment of additional search fees.

Form PCT/ISA/210 (continuation of first sheet (1)) (July 1992)